(19) Europäisches Patentamt
European Patent Office
Office européen des brevets

(11) Publication number: 0 009 728 A1

# (12) EUROPEAN PATENT APPLICATION

(21) Application number: 79103565.2

(22) Date of filing: 21.09.79

(51) Int. Cl.³: A 61 K 31/195
C 07 C 101/24, C 07 C 103/48
C 07 C 125/065, C 07 C 143/78

(30) Priority: 23.09.78 JP 116938/78
23.09.78 JP 116939 78
07.05.79 JP 55891/79

(43) Date of publication of application:
16.04.80 Bulletin 80/8

(84) Designated Contracting States:
DE FR NL

(71) Applicant: Hokuriku Pharmaceutical Co.,Ltd
1-Chome, 3-14 Tatekawacho Katsuyamashi
Fukui(JP)

(71) Applicant: Kosuge, Takuo
1-chome 33-13 Oshika Shizuokashi
Shizuocka(JP)

(72) Inventor: Kosuge, Takuo
1-chome 33-13 Oshika Shizuokashi
Shizuoka(JP)

(72) Inventor: Yokota, Masami
200-16 Nase Shizuokashi
Shizuoka(JP)

(72) Inventor: Aono, Setsuko
4-chome 23-5 Ninomiya Fukuishi
Fukui(JP)

(72) Inventor: Itoh, Yasuo
3-chome 11-14 Motomachi Katsuyamashi
Fukui(JP)

(72) Inventor: Kato, Hideo
1-chome 11-27 Motomachi Katsuyamashi
Fukui(JP)

(74) Representative: Werner, Hans Karsten, Dr. et al,
Deichmannhaus
D-5000 Köln 1(DE)

## (54) Hemostatic and platelet increasing agent containing alpha, beta-diaminopropionic acid and derivatives thereof.

(57) Hemostatic and platelet increasing agent composition comprising α,β-diaminopropionic acid and derivatives thereof represented by the formula:

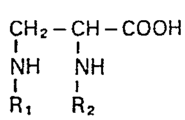

wherein $R_1$ and $R_2$ each represent a hydrogen atom, an acyl group, a benzyl group or a substituted sulfonyl group, and pharmaceutical acceptable salts thereof.

EP 0 009 728 A1

BAD ORIGINAL

# HEMOSTATIC AND PLATELET INCREASING AGENT CONTAINING α, β-DIAMINOPROPIONIC ACID AND DERIVATIVES THEREOF

The present invention relates to pharmaceutical compositions containing as an active ingredient α, β-diaminopropionic acid or derivatives thereof.

A process for preparing α, β-diaminopropionic acid which comprises treating 2,3-dichloropropionic acid with ammonia was reported in 1894 (Z. Physiol. Chem., vol. 19, page 314 (1984)). Since then several reports on the preparation thereof were made, and, in addition, an effect of inhibiting growth of microorganisms was reported with respect to its salts (Merck Index, 9th edition, page 391 (1941)).

As a result of extensive investigations on Dencichi (Panax notoginseng E. H. Chen) having hemostatic activity, the present inventors have found that L-N-β-oxalyl-α, β-diaminopropionic acid contained therein is a hemostatic active ingredient and at the same time exhibits platelet-increasing activity. This compound was given a name of "Dencichin" by the present inventors.

That is, L-N-β-oxalyl-α, β-diaminopropionic acid provides a good hemostatic activity and platelet increasing activity by the

use in an extremely small dosage and does not exhibit neurotoxic side effect which would be encountered by the use of a relatively large dose. In addition, D-form of Dencichin also possesses pharmaceutical activities similar to naturally occurring L-form. Further, the D-form is slighter in neurotoxic activity than the L-form. It has also been found that even L-α, β-diaminopropionic acid itself which is to be called as a parent substance of L-N-β-oxalyl-α, β-diaminopropionic acid exhibits hemostatic activity as well as platelet increasing activity, which activity being comparable with those of L-N-β-oxalyl-α,β-diaminopropionic acid, and provides toxicity further less than that of the oxalyl derivative by about 1/5.

That is, a rate of shortening bleeding time (hereafter referred to as "shortened rate") of the parent acid shows 44%, in contrast to 35% with the oxalyl form and its D-form, respectively which is a marked hemostatic activity.

In addition, L-α,β-diaminopropionic acid shows the increase of the number of platelet by about 17% in platelet increasing activity, as compared to a control.

That is, an object of the present invention is to provide a hemostatic and platelet increasing agent comprising α,β-diaminopropionic acid and derivatives thereof, represented by the formula:

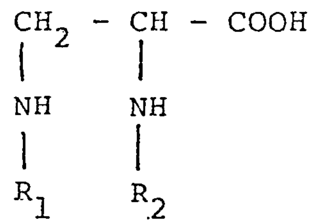

(I)

wherein $R_1$ and $R_2$ each represents a hydrogen atom, an acyl group, a benzyl group or a substituted sulfonyl group, and pharmaceutically acceptable acid salts.

The active ingredient of the present invention can shorten bleeding time as 6 to 10 times as rapid, as compared to transamine which is known as a hemostatic agent,as stated above. In particular, compounds of the formula wherein $R_1$ and $R_2$ each represents acetyl, oxalyl and benzyl provide extremely marked hemostatic activity.

$\alpha$, $\beta$-Diaminopropionic acid and derivatives thereof represented by the formula (I) are employed as active ingredients of hemostatic and platelet increasing agents as they are, or in the form of pharmaceutically acceptable salts, such as, salts of hydrochloric acid, sulfuric acid or hydrobromic acid, or salts of organic acids such as maleic acid, fumaric acid, citric acid, oxalic acid, etc. The hemostatic and platelet increasing agent containing the active ingredient of the present invention can be in the form of conventional preparations such as internal use preparations, e.g., tablet, powders, capsule, etc., injections, or external use preparations, in a conventional manner, and thus can be orally or externally administered. The dosage generally varies depending

upon symptom, manner of administration, etc., but generally is in a range of 1 to 2 mg. one time daily to achieve desired effects.

The hemostatic and platelet increasing agent of the present invention is effective not only for bleeding but also for treatment of diseases such as thrombopathy, throboasthenia, purpura haemorrhagica thrombopania, etc.

Most of α, β-diaminopropionic acid and derivatives thereof represented by the formula(I) are known compounds but some of which have not been recited in publication. These new compounds are, e.g., D-N-β-oxalyl-α, β-diaminopropionic acid, N-α-benzyl-α, β-diaminopropionic acid, etc. These α, β-diaminopropionic acid derivatives are generally synthesized using as a starting material aspargic amide via the following reaction routes.

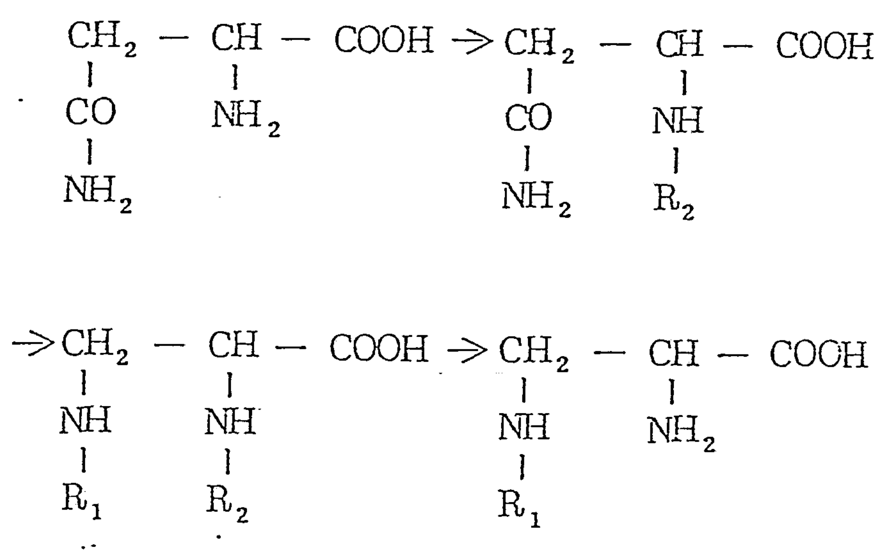

wherein $R_1$ and $R_2$ have the same meanings as defined above.

BAD ORIGINAL

In order to selectively introduce substituents at the alpha-amino group, the amino group at the beta-position is previously protected by conventional amino-protective groups such as benzyloxycarbonyl and then desired groups are introduced at the alpha-amino group in conventional manner, and the amino protective group is finally eliminated in conventional manner, for example, by stirring at room temperature together with 36% hydrobromic acid and acetic acid when benzyloxycarbonyl protective group is to be removed. These reactions are shown below, using as a representative substituent a benzyl group.

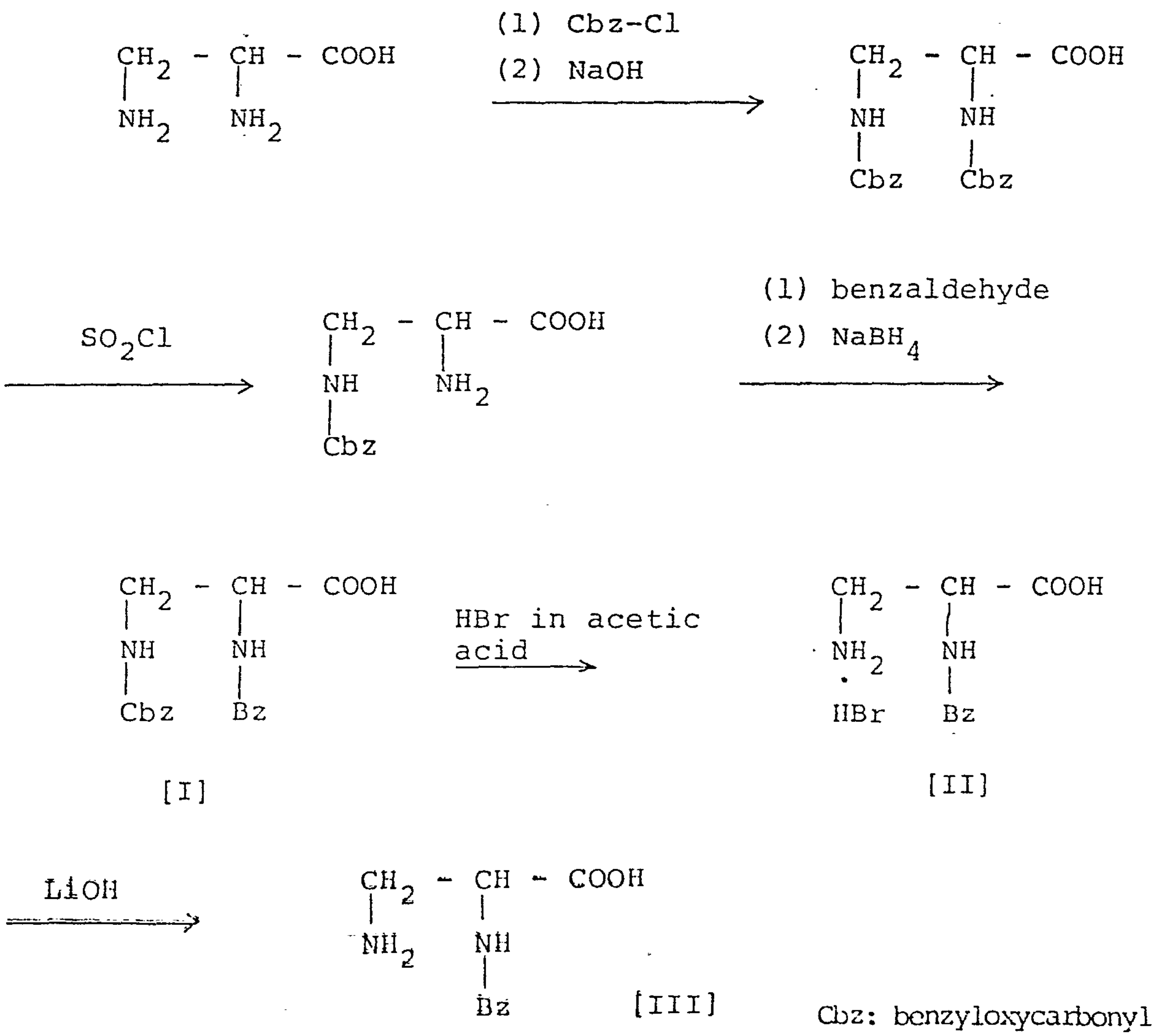


[I]

[II]

[III]

Cbz: benzyloxycarbonyl

Bz: benzyl

In the reaction scheme above, the route of [I] - [II] - [III] is shown below as synthesis examples (the procedures for preparing [I] from , -diaminopropionic acid are known).

Synthesis Example 1

α-Benzylamino-β-benzyloxycarbonylaminopropionic acid[I]:

α-Amino-β-benzyloxycarbonylaminopropionic acid (2.6 g) was dissolved in 2N NaOH aqueous solution. To the solution, 3 g. of benzaldehyde was added. After adding 300 mg. of $NaBH_4$ to the mixture small by small at temperatures lower than 5°C., the mixture was stirred for 2 hrs. The reaction liquid was extracted with ether. The water soluble portion was rendered acidic with HCl and the formed crystals were collected and recrystallized from methanol-water. [I] showing a melting point of 210°C was almost quantitatively obtained.

Synthesis Example 2

α-Benzylamino-β-aminopropionic acid·hydrobromide [II]:

A solution of 1 g. of [I] in 36% HBr and acetic acid was stirred at room temperature for 1.5 hr. To the reaction liquid, 20 ml. of dry ether was added and the formed powders were collected and dissolved in methanol. After purifying with dry ether, [II] showing a melting point of 195°C. was obtained in a good yield.

Synthesis Example 3

α-Benzylamino-β-aminopropionic acid[III]:

A solution of 500 mg. of [II] in a 5% LiOH aq. solution was stirred at room temperature for 1 hr. Then water was removed by distillation and the residue was dissolved in methanol. After purifying by adding ether thereto, [III] showing a melting point of 212°C was almost quantitatively obtained.

Further details of synthesis and reaction conditions are described in Acta Chem. Scand., vol. 14, page 961 (1960), Chem. Communs., vol. 25, page 2022 (1960) and J. Org. Chem., vol. 41, page 159 (1976), and the compounds of the formula(I) can be prepared in accordance with or by modifying the teachings of the articles above.

The present invention will be described in more detail with reference to the examples below, in which hemostatic test was performed as follows:

Hemostatic Test:

Male mice weighing 15 to 20 g. were intraperitoneously given with 0.5 ml. of a Locke solution. The mice were holded on a mouse holder 10 minutes after the administration, where the left vein of the tail at the middle of the tail (3 to 4 cm. from the root of the tail) was wounded with a knife to bleed. A filter paper was softly touched with the incised wound at the interval of 30 seconds and observation was made if blood was transferred onto the filter paper. Time until blood was not transferred onto the filter paper was measured and was made a bleeding time for control

Subsequently, a test compound dissolved in 0.5 ml. of a Locke solution was intraperitoneously administered to the same mice. The mice were holded onto a mouse holder 10 minutes after the administration. The right vein at the middle of the tail was wounded with a knife and a bleeding time was measured as in control described above.

A difference in bleeding time between the control and the test compound was made a shortened time for bleed-stopping.

Example 1

To 100 g. of Denshichi (Panax notoginseng E. H. chen.) powders, 200 ml. of methanol was added. The mixture was stirred at room temperature for 1 hr. to facilitate extraction. Then the extract was centrifuged to separate the precipitate from the portion soluble in methanol. 200 ml. of methanol was added to the precipitate to perform extraction in the same manner as above. The extraction was performed three times. Thereafter, 300 ml. of water was added to the resulting precipitate and the mixture was extracted while stirring at room temperature for 1 hr. The extract was subjected to centrifuge. To the precipitate, 300 ml. of fresh water was added and extraction was repeated three times as described above. Respective water soluble portions were combined.

5 mg. of the methanol-soluble portions and water-soluble portions were added to 0.5 ml. of a Lock solution, respectively.

Using samples obtained by adding 5 mg. of the methanol-soluble substance and the water-soluble substance to 0.5 ml. of a Locke solution, respectively, hemostatic test was performed in a manner described above. The results are shown in the table below.

Table 1

| | Control (min.) | Time until Bleeding stops (min.) | Shortened Time for Bleed-Stopping (min.) |
|---|---|---|---|
| Methanol-soluble portion | 8:00 | 8:00 | 0 |
| | 8:30 | 8:00 | 0:30 |
| Water-soluble portion | 7:00 | 5:30 | 1:30 |
| | 8:00 | 6:00 | 2:00 |

From the results above, it is understood that hemostatic active ingredient is not present in the methanol, but present only in the water-soluble portion.

Next, the water-soluble portion was shaked with a butanol-water (1:1) mixture to dissolve the water soluble portion. The insoluble matter was removed by centrifuge. The upper butanol layer was further extracted with water 10 times. The upper and lower layers were concentrated to dryness at 40°C under reduced pressure, respectively.

The results of hemostatic test are shown in the table below.

Table 2

| | Control (min.) | Time until Bleeding Stops (min.) | Shortened Time for Bleed-stopping |
|---|---|---|---|
| Upper layer | 8:00 | 7:00 | 1:00 |
| | 8:30 | 7:30 | 1:00 |
| Lower layer | 7:30 | 5:30 | 2:00 |
| | 7:00 | 5:00 | 2:00 |
| Insoluble Matter | 8:30 | 9:00 | - |

* Dose administered was 5 mg.

** The hemostatic test was performed in a manner as described above.

From the results above, it is understood that the hemostatic active ingredient was collectively concentrated in the lower layer. Thus, this fraction was subjected to Cephadex LH-20 treatment and then eluated with water. Hemostatic test was performed with respective eluate fractions. The results indicate that the hemostatic active ingredient was concentrated in a fraction of the eluate being 120 ml. to 150 ml. The range indicating shortened time for bleed-stopping of longer than 3.5 mins. was gathered and became 625 mg.

This 625 mg. was subjected to column chromatography using CM-Cephadex C-25. Eluated with water, crystals were deposited in a range of the eluate being 200 to 220 ml. This fraction was collected (50 mg.) and recrystallized from water to give 16 mg. of slender square column like crystals. The substance showed a melting point of 208°C and $[\alpha]_D^{24} = -17°$ (c=2, 4N HCl). From results of various analyse, it was found that this substance possessed the following structure.

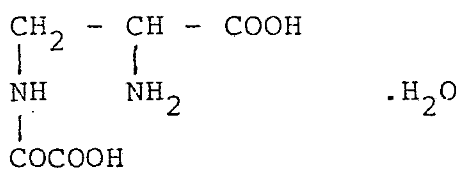

Elemental Analysis for $C_5H_8N_2O_5 \cdot H_2O$

| | C | H | N |
|---|---|---|---|
| Calcd. | 30.93 | 5.19 | 14.43 |
| Found | 31.02 | 5.21 | 14.13 |

Hemostatic property of this substance was determined in a manner as described above. The results are shown in the table below.

Table 3

| Crystal/Locke soln. (mg/ml) | Control(min.) | Time until Bleeding Stops(min.) | Shortened time for Bleed-Stopping(min.) |
|---|---|---|---|
| 1/0.5 | 8:00 | 3:00 | 5:00 |
| | 9:00 | 4:00 | 5:00 |
| 0.5/0.5 | 9:00 | 5:30 | 3:30 |
| | 8:00 | 5:00 | 3:00 |
| 0.25/0.5 | 8:30 | 7:00 | 1:30 |
| | 9:00 | 8:00 | 1:00 |
| 0.1/0.5 | 8:00 | 7:30 | 0:30 |
| | 7:30 | 7:00 | 0:30 |

Example 2

Platelet-Increasing Activity:

The compound having the formula shown in Example 1 was intraperitoneously administered to ddY female ten(10) mice as one grouping at the dose of 1 mg/0.5 ml./body in the form of a Ringer-Locke solution (1 mg. of the compound was dissolved in a Ringer-Locke solution). One eyeball was isolated 10 mins. after the administration to bleed. Blood was collected and the number of platelet was measured according to a direct method (Fonio original method), details of which are described in Dsch. Z. Schr. Chir., vol. 117, page 176 (1912).

As a control group, a Ringer-Locke solution was intraperitoneously administered at the dose of 0.5 ml/body for comparison.

The results are shown in the table below.

Table 4

| | Number of Platelet(10,000/$mm^3$) |
|---|---|
| Control | 54.9 ± 3.52 |
| Compound of Invention | 69.6 ± 6.31 |

As is seen from the results above, the number of platelet increased by about 30% when using the compound of this invention.

Example 3

Synthesis of D-N-β-Oxalyl-α,β-diaminopropionic acid:

15 g. of D-3-amino-2-(p-toluenesulfonyl)aminopropionic acid was added to a solution of 20 ml. of oxalyl chloride in 250 ml. of anhydrous dioxane. The mixture was reacted at room temperature for 6 hrs. Pieces of ice were added to the reaction liquid little by little. After the reaction liquid was concentrated to dryness, an oily substance was obtained. Dichloromethane was added to the oily substance and the mixture was allowed to stand to crystallize gradually. By filtration, 15 g. of D-3-oxalylamino-2-(p-toluenesulfonyl)aminopropionic acid was obtained.

Then, 5.9 g. of this compound and 5.2 g. of phenol were dissolved in 100 ml. of glacial acetic acid containing hydrobromic acid (32%). The solution was charged in a sealed tube and reacted at 70°C for 8 hrs.

After thoroughly cooling, the contents were poured into 600 ml. of ether. The precipitate formed was colleced and washed with a small amount of ether. The resulting product was dissolved in a small amount of water to set a column of DEAE Cephadex (3 cm x 17 cm). Elution was performed firstly with water (100 ml) and then with 2.5% HCOOH. When the eluted liquid became acidic, it was shared into 100 ml. Respective fractions were spotted on a filter paper and qualitative analysis was conducted by spraying a Ninhydrin reagent to the spot. The fractions in which the Ninhydrin test showed positive were collected and allowed to stand in an ice room, whereby crystals were formed. After collecting the crystals by filtration, the mother liquor was concentrated at a low temperature and again allowed to stand in an ice room to form crystals. These crystals were collected by filtration to give the desired product in an amount of 1.5 g.

m.p. 208°C (decomposed)

$[\alpha]_D^{24}$ +17° (c=2, 4N HCl)

Elemental Analysis for $C_5H_8N_2O_5 \cdot H_2O$

| | C | H | N |
|---|---|---|---|
| Calcd. | 30.93 | 5.19 | 14.43 |
| Found | 30.81 | 5.12 | 14.27 |

Example 4

Hemostatic Activity of D-N-β-Oxalyl-α, β-diaminopropionic acid $H_2O$:

The hemostatic activity was determined in accordance with the procedure as described above.

The results are shown in the table below.

Table 5

| Compound of Example 3/Locke soln. (mg/ml) | Control (min.) | Time until Bleeding Stops (min.) | Shortened Time for Bleed-Stopping (min.) |
|---|---|---|---|
| 1/0.5 | 8:30 | 3:30 | 5:00 |
| | 9:00 | 4:00 | 5:00 |
| 0.5/0.5 | 8:30 | 5:00 | 3:30 |
| | 8:00 | 5:00 | 3:00 |
| 0.25/0.5 | 8:30 | 7:00 | 1:30 |
| | 8:00 | 7:00 | 1:00 |
| 0.1/0.5 | 8:00 | 7:30 | 0:30 |
| | 7:30 | 7:00 | 0:30 |

Example 5

Activity on Platelet-Increase:

The procedure similar to Example 3 was repeated except that the compound of Example 3 was used instead of the compound of Example 1.

The results are shown in the table below.

Table 6

| | Number of Platelet(10,000/$mm^3$) |
|---|---|
| Control | 54.9 ± 3.52 |
| Compound of Example 3 | 68.0 ± 7.35 |

From the results above, it is clearly seen that the compound of Example 3 increases the number of platelet by approximately 24% and provides superior platelet-increasing activity.

Example 6

Hemostatic Activity:

The hemostatic activity of the compounds of the present invention was determined in accordance with the procedure described above.

The results are shown in the table below, wherein time until bleeding stopped (second) and shortened time for bleed-stopping (second) are shown by a mean value of the results obtained by repeating the test five times, together with a shortened rate by percentage when used as test compounds the compound of Example 1 and α,β-diaminopropionic acid, and, for comparison, when used a Locke solution per se and when administered none.

Table 7

| Test Compound | Bleeding Time (mean+S.E.) Before Administration | After Administration | Shortened Time (mean±S.E.) | Shortened Rate (%) |
|---|---|---|---|---|
| none | 540±21 | 534±26 | 6±22 | 1·11 |
| Locke soln. | 552±24 | 534±48 | 18±26 | 3·26 |
| L-N-β-oxalyl-α,β-diaminopropionic acid (compound of Example 1) | 546±17 | 354±45 | 192±36 | 35·16 |
| α,β-diaminopropionic acid | 546±29 | 306±37 | 240±33 $p < 0.05$ | 43·96 |